# EUROPEAN PATENT SPECIFICATION

(11) **EP 0 585 660 B1**
(45) Date of publication and mention of the grant of the patent: **03.05.2000**
(21) Application number: 93112737.7
(22) Date of filing: 09.08.1993
(51) Int. Cl.: C12Q 1/68, C12Q 1/44, C12N 9/12, C12N 9/16, C12P 19/34

(54) **Exonuclease decontamination method**
Exonuklease-Dekontaminierungsverfahren
Procédé de décontamination utilisant d'exonuclease

(30) Priority: 17.08.1992 US 931241
(43) Date of publication of application: 09.03.1994
(73) Proprietor: Becton, Dickinson and Company, Franklin Lakes, New Jersey 07417-1880 (US)
(72) Inventor: Walker, George Terrance, Chapel Hill, North Carolina 27514 (US); Fraiser, Melinda S., Durham, North Carolina 27704 (US); Schram, James L., Knightdale, North Carolina 27545 (US)
(74) Representative: Rambelli, Paolo

(56) References cited:
- WO-A-92/00384
- NUCLEIC ACIDS RESEARCH. vol. 19, no. 9 , May 1991 , ARLINGTON, VIRGINIA US page 2511 ZHU, Y ET AL 'The use of exonuclease III for polymerase reaction sterilization'
- NUCLEIC ACIDS RESEARCH. vol. 19, no. 11 , June 1991 , ARLINGTON, VIRGINIA US pages 3139 - 3141 LI, H. ET AL. 'Eliminating primers from completed polymarase chain reactions with exonuclease VII'

## Description

### Field of the Invention

The present invention relates to a method of decontaminating the products of a nucleic acid amplification reaction (amplicons) from a nucleic acid sample with exonucleases.

### Background of the Invention

DNA amplification reactions such as strand displacement amplification or polymerase chain reaction (PCR) can become contaminated with the amplification products, or "amplicons", of previous amplification reactions. Such contaminating amplicons can in turn contaminate new samples entering the lab, leading to a false positive indication of the substrate to be detected in the contaminated sample (i.e., an incorrect diagnosis of infection). Accordingly, there is a need for techniques of decontaminating, cleaning, or "sterilizing" substrate nucleic acid preparations of such amplicons.

R. Griffis, PCT Application WO 91/00363 (published 10 January 1991) discloses a method of decontaminating a PCR reaction product with a 5' lambda exonuclease Y. S. Zhu et al., Nucleic Acids Res. 19, 2511 (1991), discloses the use of exonuclease III for removing amplicons from a PCR reaction product. Both the lambda exonuclease and exonuclease III are double-strand specific exonucleases.

H. Li et al., Nucleic Acids Res. 19, 3139 (1991) discloses a method of eliminating PCR primers from a PCR reaction product with exonuclease VII. Li et al. is not concerned with the elimination of amplicons, which are of greater length than mere primers.

WO-A-92/00384 describes an improvement to the method of *in vitro* enzimatic amplification (PCR method) of a target DNA sequence present in heterologous DNA in a medium which comprises a DNA polymerase and a primer oligonucleotide. The method includes various cyclically repeated amplification steps whereby, at a chosen time following the end of the amplification cycles, the resulting amplification products are made unsuitable for later reamplification and/or, before the start of the PCR reaction, a pretreatment is carried out to selectively prevent any reamplification of the previous amplification products resulting from a PCR reaction using the same primer oligonucleotides.

### Summary of the Invention

A first aspect of the present invention is an amplicon decontamination method. The method comprises combining a nucleic acid preparation containing target nucleic acid, which preparation is potentially contaminated with nucleic acid amplification reaction products (amplicons), with a single-strand specific exonuclease, and incubating the preparation with the exonuclease for a time sufficient to degrade the amplicons. The incubating step is optionally (but preferably) followed by the step of carrying out a subsequent nucleic acid amplification reaction with the substrate nucleic acid, wherein the amplicons, if present in the preparation, would serve as a substrate for the subsequent nucleic acid amplification reaction.

In a preferred embodiment of the foregoing, the amplicons are the products of a strand displacement amplification reaction (SDA amplicons). Such SDA amplicons are characterized by the presence of phosphorothioate linkages therein.

### Detailed Description of the Invention

Nucleotide sequences are presented herein by single strand only, in the 5' to 3' direction, from left to right. One letter nucleotide symbols used herein have their standard meaning in the art in accordance with the recommendations of the IUPAC-IUB Biochemical Nomenclature Commission.

Amplification of a selected, or target, nucleic acid sequence which result in the production of amplicons may be carried out by any suitable means. See generally D. Kwoh and T. Kwoh, Am. Biotechnol. Lab. 8, 14-25 (1990). Examples of suitable amplification techniques include, but are not limited to, polymerase chain reaction, ligase chain reaction, strand displacement amplification, transcription- based amplification (see D. Kwoh et al., Proc. Natl. Acad Sci. USA 86, 1173-1177 (1989)), self-sustained sequence replication (or "3SR")(see J. Guatelli et al., Proc. Natl. Acad. Sci. USA 87, 1874-1878 (1990)), the Qβ replicase system (see P. Lizardi et al., BioTechnology 6, 1197-1202 (1988)), nucleic acid sequence-based amplification (or "NASBA")(see R Lewis, Genetic Engineering News 12 (9), 1 (1992)), the repair chain reaction (or "RCR")(see R. Lewis, supra), and boomerang DNA amplification (or "BDA")(see R. Lewis, supra). Strand displacement amplification (or "SDA"), is preferred. Various amplification procedures are, for illustrative purposes, explained in greater detail below.

Subsequent nucleic acid amplification reactions performed on nucleic acid preparations decontaminated by the method of the present invention may be carried out by any suitable amplification reaction as described above, with SDA again preferred.

Strand displacement amplification (SDA) may be carried out in accordance with known techniques. See generally G. Walker et al., Proc. Natl. Acad. Sci. USA 89, 392-396 (1992); G. Walker et al., Nucleic Acids Res. 20, 1691-1696 (1992). For example, SDA may be carried out with a single amplification primer or a pair of amplification primers, with exponential amplification being achieved with the latter. In general, SDA amplification primers comprise, in the 5' to 3' direction, a flanking sequence (the DNA sequence of which is noncritical), a restriction site for the restriction enzyme employed in the reaction, and an oligonucleotide sequence (e.g., an oligonucleotide probe of the present invention) which hybridizes to the target sequence to be amplified and/or detected. The flanking sequence, which serves to facilitate binding of the restriction enzyme to the recognition site and provides a DNA polymerase priming site after the restriction site has been nicked, is preferably about 15 to 20 nucleotides in length; the restriction site is functional in the SDA reaction (i.e., phosphorothioate linkages incorporated into the primer strand do not inhibit subsequent nicking--a condition which may be satisfied through use of a nonpalindromic recognition site); the oligonucleotide probe portion is preferably about 13 to 15 nucleotides in length.

SDA is carried out with a single amplification primer as follows: a restriction fragment (preferably about 50 to 100 nucleotides in length and preferably of low GC content) containing the sequence to be detected is prepared by digesting a DNA sample with one or more restriction enzymes, the SDA amplification primer is added to a reaction mixture containing the restriction fragment so that a duplex between the restriction fragment and the amplification primer is formed with a 5' overhang at each end, a restriction enzyme which binds to the restriction site on the amplification probe (e.g., HincII) is added to the reaction mixture, an exonuclease deficient DNA polymerase (e.g., an exonuclease deficient form of E. coli DNA polymerase I, see V. Derbyshire, Science 240, 199-201 (1988)) is added to the reaction mixture, and three dNTPs and one dNTP[∝S], with the dNTP[∝S] selected so that a phosphorothioate linkage is incorporated into the primer strand at the restriction site for the particular restriction enzyme employed (e.g., dGTP, dCTP, dTTP, and dATP[∝S] when the restriction enzyme is HincII) are added to the reaction mixture. The DNA polymerase extends the 3' ends of the duplex with the dNTPs to form a downstream complement of the target strand, the restriction enzyme nicks the restriction site on the amplification primer, and the DNA polymerase extends the 3' end of the amplification primer at the nick to displace the previously formed downstream complement of the target strand. The process is inherently repetitive because the restriction enzyme continuously nicks new complementary strands as they are formed from the restriction site, and the DNA polymerase continuously forms new complementary strands from the nicked restriction site.

SDA can also be carried out with a pair of primers on a double stranded target DNA sequence, with the second primer binding to the 3' end of the complementary strand, so that two sets of repetitive reactions are occurring simultaneously, with the process proceeding exponentially because the products of one set of reactions serve as a target for the amplification primer in the other set of reactions.

The step of first digesting the DNA sample to form a restriction fragment in an SDA reaction can be eliminated by exploiting the strand displacing activity of the DNA polymerase and adding a pair of "bumper" primers which bind to the substrate at a flanking position 5' to the position at which each amplification primer binds. Each bumper primer extension product displaces the corresponding amplification primer extension product. Amplification primers, which are present in excess, then bind to the displaced primer extension products, and upon extension, a double-stranded DNA fragment is formed which can then serve as a substrate for exponential SDA with that pair of amplification primers.

Polymerase chain reaction (PCR) may also be carried out in accordance with known techniques. See, e.g., U.S. Patents Nos. 4,683,195; 4,683,202; 4,800,159; and 4,965,188 (the disclosure of all U.S. Patent references cited herein are to be incorporated herein by reference). In general, PCR involves, first, treating a nucleic acid sample (e.g., in the presence of a heat stable DNA polymerase) with one oligonucleotide primer for each strand of the specific sequence to be detected under hybridizing conditions so that an extension product of each primer is synthesized which is complementary to each nucleic acid strand, with the primers sufficiently complementary to each strand of the specific sequence to hybridize therewith so that the extension product synthesized from each primer, when it is separated from its complement, can serve as a template for synthesis of the extension product of the other primer, and then treating the sample under denaturing conditions to separate the primer extension products from their templates if the sequence or sequences to be detected are present. These steps are cyclically until the desired degree of amplification is obtained. Detection of the amplified sequence may be carried out by adding to the reaction product an oligonucleotide probe capable of hybridizing to the reaction product (e.g., an oligonucleotide probe of the present invention), the probe carrying a detectable label, and then detecting the label in accordance with known techniques.

Ligase chain reaction (LCR) is also carried out in accordance with known techniques. See, e.g., R. Weiss, Science 254, 1292 (1991). In general, the reaction is carried out with two pairs of oligonucleotide probes: one pair binds to one strand of the sequence to be detected; the other pair binds to the other strand of the sequence to be detected. Each pair together completely overlaps the strand to which it corresponds. The reaction is carried out by, first, denaturing (e.g., separating) the strands of the sequence to be detected, then reacting the strands with the two pairs of oligonucleotide probes in the presence of a heat stable ligase so that each pair of oligonucleotide probes is ligated together, then separating the reaction product, and then cyclically repeating the process until the sequence has been amplified to the desired degree. Detection may then be carried out in like manner as described above with respect to PCR.

In general, the nucleic acid preparation employed in carrying out the present invention will be an aqueous preparation containing the target nucleic acid and any contaminating amplicons, with either (or both) the target nucleic acid and the amplicons in single-stranded form. For example, the target nucleic acid may comprise randomly sheared genomic DNA fragments. The preparation will be in a form suitable for use in a nucleic acid amplification procedure, such as those described above, in accordance with known techniques. Target nucleic acid will typically comprise nucleic acid fragments of from about 5,000 nucleotides in length to about 200,000 nucleotides in length (with lengths representing an average of the lengths found in the preparation). Preferably, the target nucleic acid is at least about 10,000 nucleotides in length, and is more preferably at least about 20,000 nucleotides in length. Within the target nucleic acid is the sequence of interest to be amplified (from hereon to be referred to as "substrate nucleic acid"). The sequences for amplification can range from as few as ten base pairs to several thousand, with base pairs of about 15 to about 200 preferred. The most preferable is a sequence of about 50 base pairs to about 100 base pairs.

The length of amplicons to be degraded by the method of the present invention will vary depending upon the particular nucleic acid amplification method by which the amplicons are produced, but will usually be at least about 25 nucleotides in length, and typically will be not more than about 2,000 nucleotides in length. When the amplicons are produced by strand displacement amplification, they will typically be not more than about 200 nucleotides in length. Amplicons and target are attacked, but due to the short length of the amplicons and their lack of secondary structure, the amplicons are preferentially cleaved.

Any single strand-specific exonuclease can be employed in carrying out the present invention so long as it is capable of degrading the amplicons. Single-strand exonucleases can be used in the absence of primers. This is particularly advantageous due to the fact that double- strand exonucleases require the presence of primers during the exonuclease digestion step in order to provide double- stranded ends on the amplicons. In addition, many double strand specific exonucleases do not completely digest the ends of amplicons because the double-stranded primer\amplicon region does not extend far enough to accommodate the binding region of the exonuclease (i.e., the exonuclease's "foot print"). And, as specifically relating to SDA, many double-strand specific exonucleases do not digest DNA containing phosphorothioate linkages (e.g., exonuclease III (Proc. Natl. Acad. Sci. 78:7350)). SDA amplicons contain phosphorothioate linkages which are cleaved by exonuclease I and exonuclease VII. Examples of suitable single-strand specific exonucleases include, but are not limited to, exonuclease VII (see, e.g., J. Chase and C. Richardson, J. Biol. Chem. 249, 4545-4552 (1974); J. Chase and C. Richardson, J. Biol. Chem. 249, 4553-4561 (1974)), exonuclease I (see, e.g., R. Brody, Biochemistry 30, 7072-7080 (1991)), Pfu DNA polymerase from Pyrococcus furiosus (Stratagene, Lajolla, CA), DNA polymerase I from E. coli, klenow fragment from DNA polymerase I of E. coli, T7 DNA polymerase, T4 DNA polymerase, spleen exonuclease (J. Biol. Chem. 253:424 (1978), T5 D15 Exonuclease (Nucleic Acids Research 19:4127 (1991), "Vent" DNA polymerase from Thermocuccus litoralis (New England Biolabs, Beverly, MA), and DNA polymerases which have 3'-5' exonuclease activity. DNA polymerases having 3'-5' exonuclease activity employed in carrying out the invention should be capable of degrading phosphorothioate linkages if the amplicons to be degraded are the products of SPA. See generally F. Eckstein. Ann. Rev. Biochem. 54, 367-402 (1985)(3'-5' exonuclease activity of T4 DNA polymerase can cleave phosphorothioate DNA but that from E. coli DNA polymerase I can not). It will be appreciated that the exonuclease need only degrade the amplicons sufficiently so that the amplicons will not serve as a substrate for a subsequent nucleic acid amplification reaction (i.e., produce a false positive result from a nucleic acid preparation which would not otherwise serve as a substrate for the amplification reaction but for contamination by the amplicons).

In practice, in a laboratory or sample believed to be highly contaminated with amplicons, every nucleic acid sample which enters the laboratory to be used in an amplification reaction which would be adversely affected by contamination with such amplicons could be decontaminated by the method of the present invention. In the alternative, if the laboratory is not believed highly contaminated by amplicons, nucleic acid samples could be split into two (or more) samples upon entry into the laboratory, with one sample being tested without decontamination and the other sample being stored. If the sample tested provides a positive result, then the stored sample could be decontaminated by the method of the present invention and then tested for the same target nucleic acid to verify that the positive result is correct, and not a false positive caused by the presence of contaminating amplicons in the sample.

It is sometimes necessary to remove amplicons from a nucleic acid sample which has itself previously been amplified. While it is preferred that the present invention be employed with nucleic acid samples which have not previously been amplified (i.e., is free of nucleic acid amplification primers), the decontamination method of the present invention could also be used on previously amplified samples although efficiency may drop.

The effectiveness of amplicon decontamination depends upon (1) the specific type of single-strand specific exonuclease, (2) the amount of exonuclease, (3) the duration of exonuclease treatment, (4) the reaction conditions (e.g., pH, salt and temperature), and (5) the relative lengths of the contaminating amplicon and the fragment that contains the target sequence. Each system can routinely be empirically optimized by varying the above variables and determining those conditions which most efficiently destroy contaminating amplicons while retaining sufficient levels of target sequence for subsequent amplification.

The present invention is explained in greater detail in the following Examples. In the Examples, µg means micrograms, µL means microliters, mL means milliliters, mM means millimolar, nM means nanomolar, v/v means volume/volume, cpm means counts per minute, and temperatures are given in degrees Centigrade unless otherwise indicated. These Examples are for illustrative purposes only, and are not to be taken as limiting of the invention. All samples in the following examples contained human placental DNA and/or calf thymus DNA in an attempt to mimic clinical M. tb samples which tend to contain human DNA.

### EXAMPLE 1

### Amplicon Products from a Previous Strand Displacement Amplification (SDA) Reaction

An SDA reaction was performed in accordance with known techniques, see G. Walker et al., Nucleic Acids Res. 20, 1691-1696 (1992), using genomic target DNA from Mycobacterium tuberculosis (M. tb) prepared in accordance with known techniques. See S. Visuvanathan et al., J Microbiol. Methods 10, 59 (1989). A 50 µL sample containing the following reagents was assembled: 50 mM K₂PO₄, pH 7.6; 100 µg/mL bovine serum albumin; 1 mM each dGTP, dCTP, TTP dATP∝S; 3% (v/v) 1-methyl 2-pyrrolidinone 6 mM MgCl₂; 5000 genome copies of DNA from M. tb; 0.25 µg of human placental DNA; primer #1 to 500 nM (SEQ ID NO:1)
primer #2 to 500 nM (SEQ ID NO:2); primer #3 to 50 nM (SEQ ID NO:3); and primer #4 to 50 nM (SEQ ID NO:4).

The sample was heated 2 minutes at 95° C followed by 2 minutes at 37° C. SDA proceeded 2 hours at 37° C after addition of 5 units of exo- klenow and 150 units of HincII. Genomic target DNA from M. tb DNA was amplified to a level of 10¹⁰ amplicons µL.

### EXAMPLE 2

### Decontamination of Amplicons with Exonuclease I for 22 Minutes

An array of samples containing various amounts of either genomic target DNA from Mycobacterium tuberculosis (M. tb) or amplicons (prepared in Example 1) were assembled as indicated in Table 1. Each 50 µL sample contained the following reagents: 50 mM K₂PO₄, pH 7.6
100 µg/mL bovine serum albumin; 1 mM each dGTP, dCTP, TTP dATP∝S; 3% (v/v) 1-methyl 2-pyrrolidinone; 6 mM MgCl₂
0.5 µg of human placental DNA; 0 or 18 µg of calf thymus DNA (Table 1); and M. tb genomic DNA or amplicons (Table 1).

Samples were heated 2 minutes at 95° C followed by 2 minutes at 37° C. Exonuclease I was added to samples as indicated in Table 1 followed by incubation for 22 minutes at 37° C. Exonuclease I was inactivated by heating 10 minutes at 56° C. SDA proceeded 2 hours at 37° C upon addition of: primer #1 to 500 nM (SEQ ID NO:1); primer #2 to 500 nM (SEQ ID NO:2); primer #3 to 50 nM (SEQ ID NO:3)
primer #4 to 50 nM (SEQ ID NO:4); 5 units of exo-klenow, and 150 units of HincII.

SDA products were detected by hybridization and extension of an amplicon specific ³²P-probe (SEQ ID NO: 5) followed by analysis using denaturing gel electrophoresis and quantitation of SDA products (³²P cpm). See Walker et al., supra.

In the presence of 0.5 µg of human DNA, a sample containing 1000 added amplicons produces a signal of 3030000 cpm after strand displacement amplification (Table 1). Prior treatment of an identical sample with 15 units of exonuclease I for 22 minutes results in a signal of 28700 cpm which is essentially equal to the background signal obtained for an exonuclease treated sample lacking added amplicons (25300 cpm).

**TABLE 1**

| Decontamination For 22 minutes with Exonuclease I | | | | |
|---|---|---|---|---|
| amplicons (molecules) | M.tb target DNA (genome molecules) | Exonuclease I (units) | human/calf thymus DNA (µg) | SDA product (³²P cpm) |
| 0 | 10 | 0 | 0.5 | 184000 |
| 0 | 1 | 0 | 0.5 | 28400 |
| 0 | 0 | 0 | 0.5 | 11500 |
| 0 | 10 | 15 | 0.5 | 365000 |
| 0 | 1 | 15 | 0.5 | 46400 |
| 0 | 0 | 15 | 0.5 | 25300 |
| 10⁴ | 0 | 0 | 0.5 | 3140000 |
| 10³ | 0 | 0 | 0.5 | 3030000 |
| 10² | 0 | 0 | 0.5 | 2000000 |
| 10⁴ | 0 | 15 | 0.5 | 235000 |
| 10³ | 0 | 15 | 0.5 | 28700 |
| 10² | 0 | 15 | 0.5 | 13500 |
| 10 | 0 | 15 | 0.5 | 7670 |
| 10⁴ | 0 | 0 | 18 | 2510000 |
| 10³ | 0 | 0 | 18 | 1530000 |
| 10² | 0 | 0 | 18 | 346000 |
| 10⁴ | 0 | 15 | 18 | 491000 |
| 10³ | 0 | 15 | 18 | 67100 |
| 10² | 0 | 15 | 18 | 8060 |
| 10 | 0 | 15 | 18 | 2000 |

In contrast, amplification of M. tb genomic DNA is not affected by prior exonuclease I treatment. Samples containing 10 or 1 M. tb genome molecules produce signals of 184000 and 28400 cpm. Prior treatment of identical samples actually results in an increase in signals (365000 and 46400 cpm). This signal enhancement probably derives from digestion of the human DNA in the samples. The presence of human DNA results in background amplification which competes with specific amplification of M. tb DNA (Walker et al. (1992), Proc. Natl. Acad. Sci. 89, 392-396). Exonuclease decontamination is not greatly affected by the presence of large amounts of human or calf thymus DNA. In the presence of 18 µg of calf thymus DNA, exonuclease I treatment of a sample containing 100 amplicons results in reduction of SDA signal from 346000 to 8060 cpm; a signal of 8060 cpm is below the background signal of 25300 cpm obtained for a standard negative control sample containing no added amplicons and 0.5 µg of human DNA.

### EXAMPLE 3

### Decontamination of Amplicons with Exonuclease I for 30 Minutes

An array of samples containing various amounts of either genomic target DNA from Mycobacterium tuberculosis (M. tb) or amplicons (prepared in Example 1) were assembled as indicated in Table 2. Each 50 µL sample contained the following reagents: 50 mM K₂PO₄, pH 7.6
100 /g/mL bovine serum albumin; 1 mM each dGTP, dCTP, dTTP dATP∝S; 3% (v/v) 1-methyl 2-pyrrolidinone; 6 mM MgCl₂
0.5 or 15 µg of human placental DNA (Table 2); and M. tb genomic DNA or amplicons (Table 2).

Samples were heated 2 minutes at 95° C followed by 2 minutes at 37° C. Exonuclease I was added to samples as indicated in Table I followed by incubation for 30 minutes at 37° C. Exonuclease I was inactiveated by heating 5 minutes at 56° C. SDA proceeded 2 hours at 37° C upon addition of: primer #1 to 500 nM (SEQ ID NO:1)
primer #2 to 500 nM (SEQ ID NO:2); primer #3 to 50 nM (SEQ ID NO:3); primer #4 to 50 nM (SEQ ID NO:4); 5 units of exo- klenow; and 150 units of HincII.

SDA products were detected by hybridization and extension of an amplicon specific ³²P-probe (SEQ ID NO:5) followed by analysis using denaturing gel electrophoresis and quantitation of SDA products (³²P cpm) (Walker et al., supra.).

**TABLE 2**

| Decontamination For 30 minutes with Exonuclease I | | | | |
|---|---|---|---|---|
| amplicons (molecules) | M.tb target DNA (genome molecules) | Exonuclease I (units) | human DNA (µg) | SDA product (³²P cpm) |
| 0 | 10³ | 0 | 0.5 | 700000 |
| 0 | 0 | 0 | 0.5 | 35800 |
| 0 | 10³ | 10 | 0.5 | 509000 |
| 0 | 0 | 10 | 0.5 | 20200 |
| 0 | 10³ | 25 | 0.5 | 1000000 |
| 0 | 0 | 25 | 0.5 | 31700 |
| 0 | 10³ | 10 | 15 | 165000 |
| 0 | 0 | 10 | 15 | 2200 |
| 0 | 10³ | 25 | 15 | 280000 |
| 0 | 0 | 25 | 15 | 4800 |
| 10⁵ | 0 | 0 | 0.5 | 1310000 |
| 10⁵ | 0 | 10 | 0.5 | 103000 |
| 10⁵ | 0 | 25 | 0.5 | 104000 |
| 10⁶ | 0 | 25 | 0.5 | 675000 |
| 10⁵ | 0 | 10 | 15 | 11400 |
| 10⁵ | 0 | 25 | 15 | 24400 |

In the presence of 0.5 or 15 µg of human DNA, treatment with either 10 or 25 units of exonuclease I for 30 minutes reduces amplification of 10⁵ amplicons to a level about 5 fold over background (Table 2). For example, the SDA signal from 10⁵ amplicons is reduced from 1310000 to 103000 cpm upon prior treatment with exonuclease I. A signal of 103000 cpm is about 5 fold greater than the corresponding background signal from a sample with no added amplicons (20200 cpm). Amplification of genomic M. tb DNA is not significantly affected by prior exonuclease I treatment. In the presence of 0.5 µg of human DNA, treatment of a sample containing 1000 M. tb genome molecules with 10 or 25 units of exonuclease I produces SDA signals of 509000 and 1000000 cpm compared to a single of 700000 cpm obtained without exonuclease.

### EXAMPLE 4

### Decontamination of Amplicons with Exonuclease VII

The experiments described in Examples 2 and 3 above are carried out in essentially the same manner as described therein, except exonuclease VII is substituted for exonuclease I as the single strand-specific exonuclease. Exonuclease VII is found to be approximately equally effective to exonuclease I.

### EXAMPLE 5

### Decontamination of Amplicons with the 3'-5' Exonuclease of T4 DNA Polymerase

The experiments described in Examples 2 and 3 above are carried out in essentially the same manner as described therein, except the 3'-5' exonuclease of T4 DNA polymerase is substituted for exonuclease I. The 3'-5' exonuclease is found to be approximately equally effective to exonuclease I.

### EXAMPLE 6

### Decontamination of Amplicons with Exonuclease I for 30 Minutes

An array of samples containing various amounts of either genomic target DNA from Mycobacterium tuberculosis (M. tb) or amplicons (prepared in Example 1) were assembled as indicated in Table 3 below. Each 50 µL sample contained the following reagents: 50 mM K₂PO₄, pH 7.6; 100 µg/mL bovine serum albumin; 1 mM each dGTP, dCTP, TTP dATP∝S; 3% (v/v) 1-methyl 2-pyrrolidinone; 3 mM MgCl₂;
0.1-10 µg of calf thymus DNA (Table 3); and M. tb genomic DNA or amplicons (Table 3).

Samples were heated 2 minutes at 95° C followed by 2 minutes at 37° C. Exonuclease I was added to samples as indicated in Table 3 followed by incubation for 30 minutes at 37° C. Exonuclease I was inactivated by heating 10 minutes at 56° C. SDA proceeded 2 hours at 37° C upon addition of: primer #1 to 500 nM (SEQ ID NO:1); primer #2 to 500 nM (SEQ ID NO:2); primer #3 to 50 nM (SEQ ID NO:3)
primer #4 to 50 nM (SEQ ID NO:4); 5 units of exo-klenow, 150 units of HincII, and MgCl₂ to a final concentration of 6mM.

**TABLE 3**

| Decontamination For 30 minutes with Exonuclease I | | | | |
|---|---|---|---|---|
| Amplicons | Exonuclease I | R.L.U. | Human DNA | Genomic DNA |
| 10⁵ | no | >20,000 | 0.1ug | zero |
| 10⁵ | yes | 1,519 | 0.1ug | zero |
| 10⁴ | no | >20,000 | 0.1ug | zero |
| 10⁴ | yes | 413 | 0.1ug | zero |
| 10³ | no | 14,375 | 0.1ug | zero |
| 10³ | yes | 124 | 0.1ug | zero |
| 10² | no | 3,469 | 0.1ug | zero |
| 10² | yes | 202 | 0.1ug | zero |
| 10 | no | 475 | 0.1ug | zero |
| 10 | yes | 115 | 0.1ug | zero |
| zero | no | 58 | 0.1ug | zero |
| zero | yes | 249 | 0.1ug | zero |
| 10² | no | 1495 | 1ug | zero |
| 10² | yes | 30 | 1ug | zero |
| 10² | no | 455 | 10ug | zero |
| 10² | yes | 18 | 10ug | zero |
| zero | no | 9,710 | 0.1ug | 100 genomes |
| zero | yes | 8,645 | 0.1ug | 100 genomes |

SDA products were detected using sequence specific probes and chemiluminescent detection. The level of amplified product is indicated by luminometer signals in terms of relative light units (R.L.U.)(Table 3).

In the presence of 0.1 µg of human DNA, treatment with 15 units of exonuclease I for 20 minutes reduces amplification of ≤1000 amplicons to background levels (Table 3). In the presence of 1 or 10 µg of calf thymus DNA, exonuclease I treatment reduces amplification of 100 amplicons to background levels. Amplification of genomic target DNA from M. tb is not significantly reduced by exonuclease I treatment.

The foregoing examples are illustrative of the present invention, and are not to be construed as limiting thereof. The invention is defined by the following claims, with equivalents of the claims to be included therein.

## Claims

1. An amplicon decontamination method comprising adding a single-strand specific exonuclease to a preparation of nucleic acids which have been rendered single-stranded, and reacting the exonuclease with the nucleic acids under conditions suitable for digestion of the nucleic acids such that the amplicons will not serve as a substrate for nucleic acid amplification.

2. A method of Claim 1 wherein the nucleic acid amplification is selected from the group consisting of polymerase chain reaction, ligase chain reaction, strand displacement amplification, transcription based amplification, self-sustained sequence replication, Qβ replicase amplification, nucleic acid sequence-based amplification, repair chain amplification, and boomerang DNA amplification.

3. A method according to Claim 1 wherein the exonuclease is exonuclease VII.

4. A method according to Claim 1, wherein the exonuclease is exonuclease I.

5. A method according to Claim 1 wherein the exonuclease is a DNA polymerase having 3'-5' exonuclease activity.

6. An amplicon decontamination method comprising adding a single-strand specific exonuclease to a preparation of nucleic acids which have been rendered single-stranded, and reacting the exonuclease with the nucleic acids under conditions appropriate for digestion of the nucleic acids such that the amplicons will not serve as a substrate for Strand Displacement Amplification.

7. A method of Claim 6 wherein the nucleic acid to be amplified is from about 10 base pair to about 200 base pair in length.

8. A method of Claim 7 in which the exonuclease is exonuclease VII.

9. A method of Claim 7 in which the exonuclease is exonuclease I.

10. A method of Claim 7 in which the exonuclease is a DNA polymerase having 3'-5' exonuclease activity.

## Patentansprüche

1. Amplicon-Dekontaminierungsverfahren umfassend das Hinzufügen einer Einzelstrang-spezifischen Exonuclease zu einer Präparation von Nucleinsäuren, die einzelsträngig gemacht wurden, und das Umsetzen der Exonuclease mit den Nucleinsäuren unter Bedingungen, die für den Verdau der Nucleinsäuren geeignet sind, so dass die Amplicons nicht als Substrat für eine Nucleinsäure-Amplifikation dienen.

2. Verfahren nach Anspruch 1, worin die Nucleinsäure-Amplifikation ausgewählt ist aus der Gruppe bestehend aus Polymerase-Kettenreaktion, Ligase-Kettenreaktion, Strangverdrängungs-Amplifikation, auf Transkription basierender Amplifikation, sich selbst erhaltender Sequenzreplikation, Qβ-Replicase-Amplifikation, auf Nucleinsäuresquenzen basierender Amplifikation, Reparatur-Kettenamplifikation und Bumerang-DNA-Amplifikation.

3. Verfahren nach Anspruch 1, worin die Exonuclease Exonuclease VII ist.

4. Verfahren nach Anspruch 1, worin die Exonuclease Exonaclease I ist.

5. Verfahren nach Anspruch 1, worin die Exonuclease eine DNA-Polymerase mit 3'-5'-Exonuclease-Aktivität ist.

6. Amplicon-Dekontaminierungsverfahren umfassend das Hinzufügen einer Einzelstrang-spezifischen Exonuclease zu einer Präparation von Nucleinsäuren, die einzelsträngig gemacht wurden, und das Umsetzen der Exonuclease mit den Nucleinsäuren unter Bedingungen, die für den Verdau der Nucleinsäuren geeignet sind, so dass die Amplicons nicht als Substrat für eine Strangverdrängungs-Amplifikation dienen.

7. Verfahren nach Anspruch 6, worin die zu amplifizierende Nucleinsäure eine Länge von etwa 10 Basenpaaren bis etwa 200 Basenpaaren aufweist.

8. Verfahren nach Anspruch 7, worin die Exonuclease Exonuclease VII ist.

9. Verfahren nach Anspruch 7, worin die Exonuclease Exonuclease I ist.

10. Verfahren nach Anspruch 7, worin die Exonuclease eine DNA-Polymerase mit 3'-5'-Exonuclease-Aktivität ist.

## Revendications

1. Procédé de décontamination d'amplicon , comprenant l'addition d'une exonucléase spécifique de simple brin à une préparation d'acides nucléiques qui ont été rendus simple brin, et la réaction de l'exonucléase avec les acides nucléiques dans des conditions convenant à la digestion des acides nucléiques de façon que les amplicons ne servent pas de substrat pour une amplification des acides nucléiques.

2. Procédé selon la revendication 1, dans lequel l'amplification des acides nucléiques est choisie dans l'ensemble comprenant la réaction en chaîne par polymérase, la réaction en chaîne par ligase, l'amplification par déplacement de brin, l'amplification à base d'une transcription, la réplication de séquence auto-entretenue, l'amplification par Qβ-réplicase, l'amplification à base d'une séquence d'acide nucléique , l'amplification en chaîne par réparation, et l'amplification d'ADN boomerang.

3. Procédé selon la revendication 1, dans lequel l'exonucléase est l'exonucléase VII.

4. Procédé selon la revendication 1, dans lequel l'exonucléase est l'exonucléase I.

5. Procédé selon la revendication 1, dans lequel l'exonucléase est une ADN-polymérase ayant une activité d'exonucléase 3'-5'.

6. Procédé de décontamination d'amplicon , comprenant l'addition d'une exonucléase spécifique de simple brin à une préparation d'acides nucléiques qui ont été rendus simple brin, et la réaction de l'exonucléase avec les acides nucléiques dans des conditions convenant à la digestion des acides nucléiques de façon que les amplicons ne servent pas de substrat pour une amplification par déplacement de brin.

7. Procédé selon la revendication 6, dans lequel l'acide nucléique à amplifier a une taille d'environ 10 paires de bases à environ 200 paires de bases.

8. Procédé selon la revendication 7, dans lequel l'exonucléase est l'exonucléase VII.

9. Procédé selon la revendication 7, dans lequel l'exonucléase est l'exonucléase I.

10. Procédé selon la revendication 7, dans lequel l'exonucléase est une ADN-polymérase ayant une activité d'exonucléase 3'-5'.


SEQUENCE LISTING

(1) GENERAL INFORMATION:

(i) APPLICANT: BECTON, DICKINSON AND COMPANY

(A) NAME: BECTON, DICKINSON AND COMPANY
(B) STREET: 1 Becton Drive
(C) CITY: Franklin Lakes
(D) STATE: New Jersey
(E) COUNTRY: USA
(F) ZIP: 07417-1880

(ii) TITLE OF INVENTION: Exonuclease Decontamination Method

(iii) NUMBER OF SEQUENCES: 5

(iv)COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25

(v ) CURRENT APPLICATION DATA:
(A) APPLICATION NUMBER:
(B) FILING DATE:

(2) INFORMATION FOR SEQ ID NO:1:

(i) SEQUENCE CHARACTERISTICS:
(A) LENGTH: 37 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:1:

TTGAATAGTC GGTTACTTGT TGACGGCGTA
CTCGACC 37
(2) INFORMATION FOR SEQ ID NO:2:

(i) SEQUENCE CHARACTERISTICS:
(A) LENGTH: 37 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:2:

TTGAAGTAAC CGACTATTGT TGACACTGAG
ATCCCCT 37

(2) INFORMATION FOR SEQ ID NO:3:

(i) SEQUENCE CHARACTERISTICS:
(A) LENGTH: 13 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:3:

TGGACCCGCC
AAC
13

(2) INFORMATION FOR SEQ ID NO:4:

(i) SEQUENCE CHARACTERISTICS:
(A) LENGTH: 13 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:4:

CGCTGAACCG
GAT
13

(2) INFORMATION FOR SEQ ID NO:5:

(i) SEQUENCE CHARACTERISTICS:
(A) LENGTH: 15 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:5:

CGTTATCCAC
CATAC
15